# EUROPEAN PATENT APPLICATION

(11) **EP 4 781 922 A1**
(43) Date of publication of application: **29.07.2026**
(21) Application number: 25154309.6
(22) Date of filing: 28.01.2025
(51) Int. Cl.: A61B 17/00, A61M 11/02, A61M 39/22, A61M 39/24

(54) **ASSEMBLY FOR DELIVERING SUBSTANCES THROUGH A DELIVERY CATHETER**

(71) Applicant: Maslanka Patentverwaltung GmbH, 78532 Tuttlingen (DE)
(72) Inventor: MASLANKA, Herbert, 78532 Tuttlingen (DE)
(74) Representative: Weickmann & Weickmann PartmbB

(57) **Abstract**

The present invention relates to an assembly (100) for delivering substances through a delivery catheter (102), comprising the delivery catheter (102), a connector element (104) with a body portion (106), which is adapted to be connected to a proximal end of the delivery catheter (102) at a connection portion (114) thereof, wherein the connector element (104) further comprises a first attachment section (116) which is adapted for connection with a gas delivery line (118) providing a gas flow through the catheter (102), and a second attachment section (120) which is adapted for connection with a reservoir (110) containing the substance to be delivered through the catheter (102), the assembly further comprising a gas source (108) for providing the gas flow and the reservoir (110) containing the substance. According to the present invention, it further comprises a sealing element (122) which in a closed configuration prevents the substance from being carried through the catheter (102) by the gas flow, and which in an open configuration allows the substance to be carried through the catheter (102) by the gas flow.

## Description

The present invention relates to an assembly for delivering substances through a delivery catheter, comprising the delivery catheter, a connector element with a body portion, which is adapted to be connected to a proximal end of the delivery catheter at a connection portion, wherein the connector element further comprises a first attachment section, which is adapted for connection with a gas delivery line providing a gas flow through the catheter, and a second attachment section, which is adapted for connection with a reservoir, containing the substance to be delivered through the catheter, wherein the assembly further comprising a gas source for providing the gas flow as well as the reservoir containing the substance.

It is known in the corresponding field that in medical procedures certain substances and in particular powdery or liquid substances may be used for different purposes which have to be delivered into the subject or patient of the medical procedure, in particular by means of a catheter. One particular example is the delivery of hemostatic substances in order to locally stop extensive bleeding. Such substances usually bind the water from the blood escaping from blood vessels and as such locally increase the platelet concentration, which facilitates blood coagulation.

In order to be able to transport such substances to difficult to reach locations in the body of subjects during a corresponding medical procedure, assemblies of the above-mentioned type have been used, in which the corresponding substances are first contained in a reservoir and are then delivered from said reservoir through the catheter to the location to be treated by means of a gas flow through the catheter, carrying and transporting the substance.

However, in known examples of such assemblies, the operation thereof and in particular the release and metering of the corresponding substances have been quite cumbersome, since the operator in charge of administering the substance had to manually control the mixture of the substance with the gas flow, which oftentimes required a certain orientation of the reservoir with respect to a horizontal plane, such that there was the risk of mishandling and delivering a too small or too large amount of the substance, which could negatively influence the effectiveness thereof and further pose the risk of clogging the catheter. Furthermore, there was the risk of an undesired delivery of the substance, in the worst case into the working channel of the endoscope, which might lead to a clogging of said working channel.

There is therefore a demand for an assembly for delivering substances through a delivery catheter with improved handling capabilities and ease of use for its operator.

For this purpose, the present invention proposes an assembly of the type briefly discussed above, which further comprises a sealing element, which in a closed configuration prevents the substance from being carried through the catheter by the gas flow, and which in an open configuration allows the substance to be carried through the catheter by the gas flow, or a sealing element, which is is formed in such a manner, for example slotted, that a section of the reservoir can be pushed through the sealing element, whereupon the sealing element will fit tightly around said section of the reservoir thus sealing it. Thus, by providing said sealing element, which is selectively translatable between an open and a closed configuration or said sealing element which allows for pushing a section of the reservoir therethrough for its activation, the mixing of the substance with the gas flow can easily be started and stopped, without the orientation of the reservoir with respect to the horizontal plane becoming an issue which the operator has to account for. Furthermore, the metering of the substance to be delivered becomes easier and less care has to be taken that the correct amount of the substance is being delivered to the desired location at the required timing. It shall be pointed out that in case the sealing element is formed such that a section of the reservoir can be pushed through it, it does not necessarily have to be fully gastight in its unused state, in which the reservoir is not present.

In one possible embodiment of the assembly according to the present invention, the body portion of the connector element may have a substantially tubular shape along a longitudinal direction, wherein said longitudinal direction may serve as an extension to the principal direction of the delivery catheter connected thereto. Such a tubular shape facilitates gripping and handling of the connector element during use and thus reduces the risk of operator errors.

In particular, the connection portion of the connector element may be positioned on a first end of the body portion, wherein in particular the second attachment section may be positioned on a second end of the body portion opposite to said first end. Thus, in such an embodiment of the assembly according to the present invention, the reservoir containing the substance lies opposite to the connection to the catheter and is as such aligned with it along the extension of the body portion of the connector element. This geometry also facilitates the metering of the substance to be delivered through the catheter of the assembly in that the substance may be transported in a straight line through the body portion of the connector element and thus turbulences can be avoided or reduced.

Furthermore, the first attachment section for connection with the gas delivery line may be positioned between the connector element and the second attachment element with respect to the longitudinal direction of the body element, preferably in a slanted manner towards the second attachment section. Thus, the gas flow will enter the body portion of the connector element from a side, in particular in a substantially tangential manner, such that a relatively smooth flow of the gas can be ensured in order to further reduce turbulence.

In one particular embodiment, the sealing element may be formed by a turning valve with an actuating element, wherein preferably in the open configuration the actuating element may be aligned with the longitudinal direction of the body portion of the connector element. This provides for an intuitive handling of the turning valve, wherein such types of valves may not only exhibit a single open and a single closed configuration, but rather different degrees of opening, which may further increase the precision of the delivery of the substance through the catheter. Other variants of such actuatable valves may also be used in an assembly according to the present invention, such as for example comprising sliding switches or pushers. Other possible embodiments of the sealing element comprise trumpet valves, duckbill check valves and spring valves.

In another possible embodiment, the sealing element may be formed as a check valve, which is normally in its closed configuration and only upon an activation of the reservoir transitions to its open configuration, wherein such an activation of the reservoir in particular refers to forcefully pushing the contents thereof through the check valve, which in this case will open.

Yet another variant of the present invention may further comprise a lid element which is attachable to the connector element serving as the sealing element in order to constitute the closed configuration of the sealing element and is removable to constitute the open configuration of the sealing element. Therein, the attachment of the lid element may be performed by any suitable means, for example cooperating threaded portions or a releasable snap connection.

In this context, the reservoir of the assembly may be formed in a compressible manner, for example as a bellows, in order to release the substance contained therein into the connector element, in which it will mix with the gas flow entering through the first attachment section and will subsequently be carried and transported through the catheter. The use of such a bellows also improves the handling properties of the assembly, as the operator thereof will have a tactile feedback on how the substance is dispersed, such that the correct metering of the substance is facilitated. It shall also be pointed out that such a bellows used as the reservoir also already may serve as a mixing chamber, in which the substance is dispersed into the gas contained therein, which further reduces the risk of clogging of the connector element or the catheter.

In order to further improve the handling properties of the assembly, the connector element may further comprise a handle section extending from the body portion, which an operator may grip during the use of the assembly. In particular, the operator may grip said handle section with two or more fingers and may then press the above-mentioned bellows with the palm of their hand.

It shall furthermore be pointed out that while the gas source may also be operated manually, for example in the form of a further bellows, it may also be formed by an automatic air pump device or a pressured air mains arrangement, preferably comprising an air filter. Such an air pump device may be selectively turned on and off in order to provide the air flow through the gas delivery line, the connector element and subsequently the catheter, which also improves the precision of the metering of the substance to be delivered through the catheter.

In assemblies according to the present invention, it is desirable to use a low friction and/or smooth and/or non-polar catheter material on its inner side in order to further reduce the risk of clogging of the substance therein, such that in particular PE, PP, PA, TPE or TPU material may be used, as well as fluor plastic materials, such as PTFE, PFA or FEP.

Similarly, the connector element may be made from a plastic material, preferably by means of injection molding, wherein such materials are relatively cheap and have sufficient mechanical properties to serve this purpose.

It shall further be pointed out that the substance contained in the reservoir may in particular be a powdery or liquid substance and/or a hemostatic in order to reduce bleeding in subjects during medical procedures. The reservoir may also contain a certain amount of air or another suitable gas in order to pre-disperse the substance even before it enters the connector element of the assembly.

It shall also be pointed out that in certain embodiments of the present invention, the second attachment section may be rotatable with respect to the connector element.

Further features and advantages of the present invention will become even clearer from the following description of two embodiments thereof, when viewed together with the enclosed figures. These figures show in particular:
- Figure 1: a first embodiment of an assembly according to the present invention in a partially transparent isometric view;
- Figure 2: a second embodiment of an assembly according to present invention in a similar view as figure 1; and
- Figure 3: a further variant of the embodiment shown in figure 2.

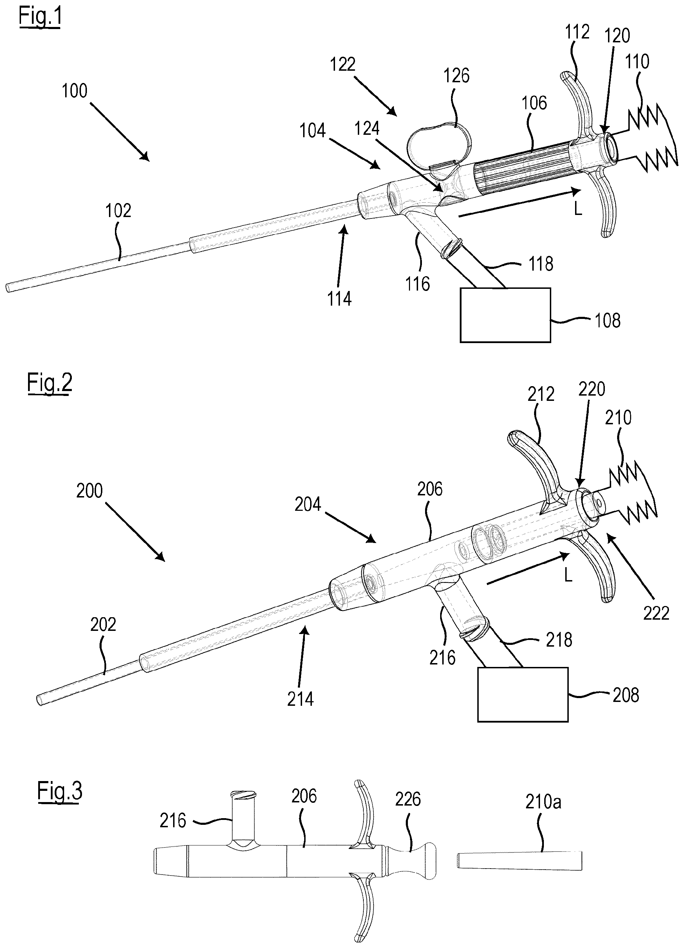

Figure 1 shows a first embodiment of an assembly for delivering substances through a delivery catheter according to the present invention, which is generally denoted with reference numeral 100. It comprises the delivery catheter 102, a connector element 104 with a body portion 106, a gas source 108, which is only shown schematically, and a reservoir 110 containing the corresponding substance.

As can be seen in figure 1, the body portion 106 of the connector element 104 is formed in a substantial tubular shape with a hollow inner space and a handle section 112 extending to both sides thereof. The body portion 106 comprises in total three interfaces, a connection portion 114, which is adapted to be connected to the proximal end of the delivery catheter 102, a first attachment section 116, which is adapted for connection with a gas delivery line 118, providing a gas flow from the gas source 108, and a second attachment section 120, which is adapted for connection with the reservoir 110. Herein, the second attachment section 120 may be formed in a rotatable manner, in order to achieve improved handling properties.

As can also be seen in figure 1, the connection portion 114 is positioned on the first end of the body portion 106 with the second attachment section 120 being positioned on the second end thereof opposite to the first end. Additionally, the first attachment section 116 is positioned between the connector portion 114 and the second attachment section 120 with respect to the longitudinal direction L of the body element 106, in a slanted manner towards the second attachment section 120.

As can be understood from figure 1, the connector element 104 further comprises a sealing element 122 in the form of a turning valve 124 with an actuating element 126 which may be turned by an operator of the assembly 100. In particular, figure 1 shows an open configuration of the sealing element 122 in which the substance from the reservoir 110 may mix with the gas flow provided through the first attachment section 116 in order to be delivered through the catheter 102 to a location inside the body of a subject of a medical procedure. By turning the actuating element by ninety degrees, the sealing element 122 can be transitioned into its closed configuration such that the substance in the reservoir 110 may no longer mix with the gas flow such that its delivery through the catheter is prevented. It shall be pointed out that all connections just described may be achieved by known suitable means, for example by screwing the corresponding components onto threads provided at the respective attachment sections of the connector element 104. The reservoir 110 may also be connected my means of a threaded connection, a luer connection or a snap connction.

Figure 2 now shows a second embodiment of an assembly according to the present invention which is generally denoted with reference numeral 200. It differs from the assembly 100 shown in figure 1 only by its type of sealing element 222 and as such a further explanation of the remaining components thereof is omitted. It shall rather be referred to the above description of the embodiment shown in figure 1 and all corresponding components identically or similarly also found in the embodiment of figure 2 are denoted with the same reference numerals, increased by 100.

As can be understood from figure 2 the corresponding sealing element 222 is formed by a proximally closed sealing element, in which the sealing element 222 is slotted, such that the reservoir 210 can be pushed through the sealing element by means of a suitably shaped insertion section 210a, which is schematically shown in figure 3 in a similar context. Hereupon, the sealing element 222 will fit tightly around the conical insertion section 210a of the reservoir 210 thus sealing it. In such an embodiment, the catheter 202 may be inserted into the endoscope while pressured air is delivered, such that no moisture may enter the channel of the endoscope, thus preventing clogging of the catheter 202 upon delivering the substance. Only after the catheter 202 has been inserted into the endoscope and has been placed at its working position, the user may push the reservoir through the sealing element.

In another variant similar to what is shown in figure 2, the sealing element is formed by a check valve which in its normal or non-operated state is in its closed configuration. In further alternative variants, other types of valves or sealings may be employed. Therefore, only upon actuation of the reservoir in form of a bellows, the air therein is compressed, the substance is pre-dispersed and the check valve will open, such that mixing of the substance contained in the reservoir with the gas flow entering the connector element through the first attachment section is possible.

Figure 3 shows yet another variant of the present invention, similar to the embodiment shown in figure 2 and therefore denoted with the same reference numerals. Therein, a lid element 226 is provided which may be attached to the connector element, thus serving as a sealing element in order to constitute the closed configuration and is removable from the sealing element to constitute the open configuration. In a similar manner as with the embodiment shown in figure 2, a conical insertion section 210a of the reservoir 210 may then by inserted into the connector element once the lid element 226 has been removed and the assembly is in its open configuration.

All of the embodiments shown in figures 1 to 3 facilitate the handling of the respective assemblies 100 and 200 according to the present invention in that a horizontal orientation of the corresponding reservoirs during use of the assemblies 100 and 200 becomes less critical, since by means of the sealing elements 122, 222, the delivery of the substance may selectively be stopped and started and the operator at all times has full control over whether substance is delivered through the catheter 102, 202 or not.

## Claims

1. Assembly (100, 200) for delivering substances through a delivery catheter (102, 202), comprising:
- the delivery catheter (102, 202);
- a connector element (104, 204) with a body portion (106, 206), which is adapted to be connected to a proximal end of the delivery catheter (102, 202) at a connection portion (114, 214) thereof,
wherein the connector element (104, 204) further comprises:
∘ a first attachment section (116, 216) which is adapted for connection with a gas delivery line (118, 218) providing a gas flow through the catheter (102, 202), and
∘ a second attachment section (120, 220) which is adapted for connection with a reservoir (110, 210) containing the substance to be delivered through the catheter (102, 202);
- a gas source (108, 208) for providing the gas flow; and
- the reservoir (110, 210) containing the substance;
**characterized in that** it further comprises a sealing element (122), which in a closed configuration prevents the substance from being carried through the catheter (102) by the gas flow, and which in an open configuration allows the substance to be carried through the catheter (102) by the gas flow, or
a sealing element (202), which is formed in such a manner that a section (210a) of the reservoir (210) can be pushed through the sealing element (202), whereupon the sealing element (202) will fit tightly around said section of the reservoir (210) thus sealing it.

2. Assembly (100, 200) according to claim 1,
wherein the body portion (106, 206) of the connector element (104, 204) has a substantially tubular shape along a longitudinal direction (L).

3. Assembly (100, 200) according to claim 1 or 2,
wherein the connection portion (114, 214) is positioned on a first end of the body portion (106, 206).

4. Assembly (100, 200) according to claim 3,
wherein the second attachment section (120, 220) is positioned on a second end of the body portion (106, 206) opposite to the first end.

5. Assembly (100, 200) according to claim 4,
wherein the first attachment section (116, 216) is positioned between the connector portion (114, 214) and the second attachment section (120, 220) with respect to the longitudinal direction (L) of the body element (106, 206), preferably in a slanted manner towards the second attachment section (120, 220).

6. Assembly (100) according to any of the preceding claims,
wherein the sealing element (122) is formed by a turning valve (124) with an actuating element (126), wherein preferably in the open configuration the actuating element (126) is aligned with the longitudinal direction (L) of the body portion (106) of the connector element (104), or
wherein the sealing element is formed by a trumpet valve, a duckbill check valve or a spring valve.

7. Assembly according to any of claims 1 to 5,
wherein the sealing element is formed as a check valve which is normally in its closed configuration and upon an activation of the reservoir transitions to its open configuration.

8. Assembly according to any of claims 1 to 5,
further comprising a lid element (226) which is attachable to the connector element serving as the sealing element in order to constitute the closed configuration and is removable to constitute the open configuration.

9. Assembly (100, 200) according to any pf the preceding claims,
wherein the reservoir (110, 210) is formed in a compressible manner, for example as a bellows, in order to release the substance contained therein.

10. Assembly (100, 200) according to any of the preceding claims,
wherein the connector element (104, 204) further comprises a handle section (112, 212) extending from the body portion (106, 206).

11. Assembly (100, 200) according to any of the preceding claims,
wherein the gas source (108, 208) is formed by an air pump device, preferably comprising an air filter.

12. Assembly (100, 200) according to any of the preceding claims,
wherein the delivery catheter (102, 202) is made from a low friction und/or smooth and/or non-polar material.

13. Assembly (100, 200) according to claim 11,
wherein the material consists of PE, PP, PA, TPE, TPU, PTFE, PFA or FEP.

14. Assembly (100, 200) according to any of the preceding claims,
wherein the connector element (104, 204) is made from a plastic material, preferably by means of injection moulding.

15. Assembly (100, 200) according to any of the preceding claims,
wherein the second attachment section (120, 220) is rotatable with respect to the connector element (104, 204).
